# EUROPEAN PATENT APPLICATION

(11) **EP 1 495 713 A1**
(43) Date of publication of application: **12.01.2005**
(21) Application number: 03015812.5
(22) Date of filing: 10.07.2003
(51) Int. Cl.: A61B 5/00

(54) **Portable medical device**

(71) Applicant: Fahad, Faisal Almoammar, 11472 Riyadh (SA)
(72) Inventor: Fahad, Faisal Almoammar, 11472 Riyadh (SA)
(74) Representative: Appelt, Christian W.

(57) **Abstract**

The invention relates to a portable medical device which comprises a detection unit for detecting at least one parameter concerning the condition of a living body, wherein it further comprises a telecommunication unit for a data and/or voice communication, the telecommunication unit being capable of transferring data to and/or of receiving data from a remote data transceiver.

## Description

The present invention relates to a portable medical device which comprises a detection unit for detecting at least one parameter concerning the condition of a living body.

The treatment of a an illness or a medical precaution to prevent an illness requires in many cases a frequent and regular detection of one or several parameters concerning the condition of a living body, such as the amount of certain substances in the blood or in the urine, the body temperature or the blood pressure. An observation of such parameters over a longer period gives in many cases a reliable indication whether the condition of a living body improves or weakens. This applies to a human body as well as to the body of an animal.

In most cases precise measurements of such parameters are only made in a doctor's office or a health center where access to a medical lab is possible. This makes an observation of parameters over a longer period difficult or even impossible since precise measurements may only be taken if the patient comes to the doctor's office or to the medical health center.

Persons having an illness where blood regularly needs to be sampled are e.g. diabetes patients. Persons suffering from type 1 diabetes need to check their blood sugar value e.g. three times a day. This is usually done by bringing a small quantity of blood in contact with a test strip which indicates, e.g. by a change of its colour, whether the blood sugar value is too high. If a too high blood sugar value is recognized, an injection of insulin is necessary. Those injections may usually be done by the persons concerned themselves. Therefore, type 1 diabetes patients usually may live relatively independent from medical assistance and do not need to visit a doctor's office too often.

Type 2 diabetes, the so-called non-insulin dependent diabetes is linked to heredity and obesity and may be controlled by a diet. The monitoring of the blood sugar value is not necessary within very short periods, i.e. every day, but from time to time in order to monitor the development of the illness or a corresponding medical treatment.

Using test strips, the monitoring of variations of a blood sugar value of a patient is not very accurate, since the change of the value is related to a change of the colour of the test strip which is very difficult to quantify. Those measurements cannot provide precise blood sugar values and are therefore not sufficient for a long-term monitoring of their timely development. Precise values usually may be determined only during a visit in a doctor's office or in a medical center making use of a medical lab.

However, continuous measurements can usually better reflect the progress of a treatment. In principle this applies for all illnesses and also for the precaution of an illness.

In order to improve the frequency and thus the quality of long-term continuous measurements patients can be provided with relatively small portable devices, which may be used at home and which have a memory such that data gained from several successive measurements can be stored. As an example for known devices of such kind portable blood pressure instruments may be mentioned.

In principle corresponding devices may be used by all different kinds of patients suffering from illnesses, where a treatment requires a frequent and regular taking of samples of body fluids, such as blood or urine, in order to monitor changes related to the illness or the corresponding medical treatment, or in the case where long-term continuous measurements of other body related parameters, such as for example the body temperature, is necessary.

If a corresponding device includes a unit for an automatic analysis of data or at least a display where measured values can be displayed, the patient may eventually decide on his own whether a certain reaction to a measurement of a parameter as for example the change of the amount of medicine is necessary. However the evaluation of medical data is usually to difficult or even impossible for the patient and with respect to the serious consequences a wrong interpretation of a measurement may have he needs to consult a doctor or at least to send him the corresponding data for a professional analysis and advise. However, this may be rather inconvenient for persons frequently travelling or living far away from a medical health center.

Therefore, it is an object of the present invention to provide a portable medical device which permits to regularly acquire data related to parameters concerning the condition of a living body, such as substances and their amount in the blood or urine, body temperature, the blood pressure, pulse, action currents of the heart etc., and which furthermore permits to transfer acquired data to a health professional to provide this person with sufficient and sufficiently precise data permitting a remote diagnosis and medical advises and which furthermore permits to receive data and/or instructions from the health professional, even if the patient is far away from a doctor's office.

This object is achieved by a portable medical device comprising the features of claim 1. According to the invention a portable medical device comprises a detection unit for detecting at least one parameter concerning the condition of a living body, such as substances and their amount contained in the blood or urine, the body temperature, the blood pressure, the pulse, the action currents of the heart etc. The invention is characterized therein, that it further comprises a telecommunication unit for a data and/or voice communication, the telecommunication unit being capable of transferring data to and/or of receiving data from a remote data transceiver.

Advantageously, the portable medical device according to the present invention includes a telecommunication unit for transferring acquired medical data to a remote receiver or a medical data bank. It thus permits to transfer data that a receiver such as a health professional or any other person needs e.g. to monitor a patient's condition, even if the patient stays far away from a treatment center or the place where a person receives the data, respectively. Data analysis permits the health professional or any other person e.g. to change and precisely adapt the amount of medicine prescribed without the necessity for the patient to travel to a personal consultation. Furthermore, sudden changes of the patient's constitution may be more easily detected and a corresponding fast reaction becomes possible. Finally medical advises and a plan for the further treatment may be transferred back to the patient. Moreover it is conceivable that the data is transferred to a means such as a computer, where data analysis and a corresponding response containing advises or instructions for a patient is automatically effected.

According an advantageous embodiment of the present invention the telecommunication unit of the portable medical device automatically transfers data to and/or receives data from said remote data transceiver. This automatic transfer may be provided, if at least one criterion is fulfilled, e.g. a certain period of time has passed or a certain number of data acquisitions is completed.

This has the advantage that data may almost continuously or even continuously be transferred to the receiver, e.g. a medical data bank or the computer of a doctor without the patient being obliged to transfer the data by himself and to remember and memorize certain dates at which a transfer of data is to be effected. Furthermore, the portable medical device may automatically receive data such as instructions, advises and also signals which may automatically control certain functions in the portable medical device from the doctor or a remote control unit, respectively.

According to a further advantageous embodiment of the invention the telecommunication unit of the portable medical device transfers data to and/or receives data from said remote data transceiver in response to an instruction of a user, wherein said instruction is entered by an input unit provided on the portable medical device.

This embodiment comprises the advantage that data such as blood values measured may be spontaneously and directly transferred to a data receiver such as a medial health center or a doctor's office. This may be important e.g. in the case that a user of the portable medical device observes a serious deviation of a measurement compared to earlier measurements and immediately wishes a consultation. Furthermore, data such as tables and general advises may also be received by the user by e.g. fetching them in a remote data bank.

Instructions may be entered by an input unit such as a keyboard or by a speech recognition unit provided in the portable medical device. This has the advantage that the user of the portable medical device may contact the doctor and may transfer e.g. blood values measured, e.g. simply by pressing a button on the key panel or by dialing a number. On the other hand, if a speech recognition unit is provided, the doctor's office may be called just by speaking the doctor's name or his phone number. This further facilitates getting in contact with the doctor and transferring data. The same advantages apply for receiving data.

According to a further embodiment the portable medical device comprises a display unit for displaying data. Providing such a display on or connected to the portable medical device has the advantage that blood data may be visually analyzed by the user. If necessary, the user may for example change, within certain boundaries the amount of medicine in dependence on values measured and displayed or may instead consult his doctor.

According to still another favorite embodiment the portable medical device includes a telecommunication unit using a mobile telephone network.

Mobile telephone networks are very widespread since a few years. Accordingly, the user of the portable medical device may transfer data from and/or receive data from any location, i.e. even from remote parts of a country, having access to a telecommunication network or telecommunication means such as a mobile telephone network. In the recent time it has become possible to use mobile telephone networks even in foreign countries. This provides the user with still more mobility.

According to still another embodiment of the portable medical device the detection unit is for example a body fluid sampling unit, a body temperature measurement device, a blood pressure measurement device, a pulse measurement device, a device for measuring action currents of the heart or the brain or a combination of two ore more of them. Thus body fluids like blood or urine may be sampled and furthermore important parameters such as the body temperature, the heart pulse or action currents of the heart or the brain may be measured.

Still another embodiment provides that the portable medical device comprises a medical application unit. This is of advantage, because it is simpler for the user to have all functions like data acquisition or analyzing, transfer of data to a doctor, reception of instructions and/or advises and even medication combined in one single device. The application unit may be, according to a further embodiment, a syringe, which may e.g. be used for the injection of insulin.

According to yet another preferred embodiment the medical application unit automatically provides a prescribed amount of medicine either in response to one or several parameters detected by the detection unit or in response to an instruction received from a remote data transceiver or in response to an instruction entered by the user. This has the advantage that a patient is relieved from the burden of determining the amount of a prescribed medicine which may be difficult if it is e.g. stored in a container storing several doses onboard the portable medical device and the user does not have any measurement instrument like a graduated beaker etc.

According to still a further preferred embodiment the portable medical device is integrated into a mobile phone. Thus, the portable medical device comprises all advantages of mobile phones such as small size, high mobility and access to telecommunication networks at almost all places.

The invention will be better understood and further details and features thereof will become apparent from the following description which in combination with the appended drawing describes a preferred embodiment of the invention.
- Fig. 1: schematically shows a top view of the inventive portable medical device according to a preferred embodiment of the invention.
- Fig. 2: schematically shows components of the portable medical device according to an embodiment of the invention.

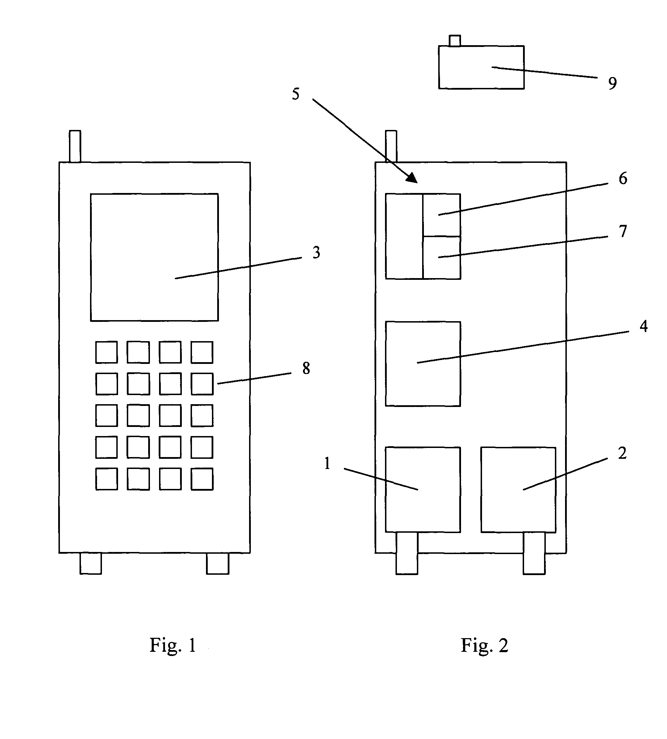

Fig. 1 represents a preferred embodiment of the portable medical device according to the present invention comprising a detection unit 1, a display unit 3 and an input unit 8 such as a keyboard or key panel. Other common means which may be used as an input unit 8 are a control ball, a control wheel, a voice recognition means, a control pin or a touch panel in combination with the display 3 and/or eventually a menu program displayed thereon. The detection unit 1 may be designed in a way that a body fluid, such as blood or urine, which is brought into contact with the detection unit 1 is automatically sampled for certain components, such as blood sugar, and/or their amount contained in the fluid. It may also be possible to analyze other body fluids such as urine or other parameters related to the human body or the body of an animal such as temperature, blood pressure, the pulse, action currents of the heart or the brain, if other corresponding devices are alternatively or additionally integrated into the portable medical device or connected therewith. It is conceivable to provide several devices combined in one detection unit 1 or to provide several detection units 1.

The result of an analysis may be stored in a data memory 4 and/or may be displayed on a display unit 3, such as a LCD, for example as a graphic chart or simply as a column of data. According to the result the user may decide whether it is necessary to take any measures such as taking a medicine or he eventually wishes to consult a doctor.

As especially shown in Fig. 2 the portable medical device furthermore comprises a telecommunication unit 5 including a transmitter 6 and a receiver 7. The telecommunication unit 5 may be used to transfer data resulting from a body fluid, blood analysis or analysis of another parameter to a remote medical data bank 9 or a data receiver, respectively, such as the computer of a health professional. The transmission of data may be effected by using for example a mobile telephone network. In order to transfer data a certain telephone number or a number corresponding to an internet site is dialed which may be stored in an internal memory 4 of the portable medical device. The number may be dialed by the user on the input unit 8 using the buttons arranged thereon or by using a program menu displayed on the display unit 3 which may also be operated via the input unit 8 or by any other device such as a control wheel, a control ball (not shown). Furthermore, a voice recognition unit may be provided permitting the user to dial a certain number of a mobile telephone network just by speaking the respective number into a microphone (not shown) provided on the portable medical device and connected to the voice recognition unit, which then automatically dials the desired telephone number. After an access for example to the internet is established, data concerning e.g. a blood analysis may be transferred to a receiver such as a health professional's office. Since the portable medical device is also equipped with a receiver data may be received as well. This data may correspond to voice or to a text including advises, instructions, a technical manual, a prescription, a dosage recommendation for a medicine etc. Those data may be received upon a transmission from a remote transmitter or by fetching it from a remote data bank.

Furthermore, the device may be designed in a way that data stored in the data memory 4 is automatically transferred to a remote medical data bank 9, for example in a doctor's office or a medical center after a certain period of time has elapsed, e.g. one day or one week, or after a certain number of data acquisitions has been effected. The portable medical device may furthermore include one or several interfaces (not shown) permitting readout of data by connecting the portable medical device to a computer or by using an infrared interface. Moreover the portable medical device may comprise further useful tools known from mobile phones such as a means for sending and/or receiving SMS messages. This feature may be used for transferring comments in relation with the data transferred to a remote medical data bank or a doctor's office without the need to directly talk to the doctor or to leave a voice mail.

Additionally, the portable medical device may also comprise a medical application unit 2. Such a unit may be a syringe, for example for an insulin injection or a cardiac pacemaker (i.e. a not implanted device). A patient may thus directly use the portable medical device for the treatment. It is however apparent that only an application unit which is sufficiently small may be housed in the device in order to keep it still portable. In the case of a larger application unit it is also conceivable to externally connect it to the portable medical device. Furthermore, a depot for a certain amount of solid medicine, i.e. a powder or pills or fluid medicine may also be provided in the portable medical device. If medicine is provided directly in the portable medical device, a fully automatic application unit can be provided. A fully automatic application unit may comprise a means being capable to measure a prescribed amount of fluid or pulverized medicine or a number of pills stored in a depot onboard the device which is then provided at an output for the user or directly introduced e.g. into a syringe and may then be injected into the body of a user. In combination with the receiver 7 provided in the portable medical device the prescribed quantity of a medicine may be received automatically by the telecommunication unit 5 and may automatically be provided by the application unit 2 without the user being charged with any handling of medicine.

The steps of using the portable medical device are described by means of the exemplifying case of a diabetes patient. A person suffering from diabetes regularly needs to take samples of his blood and analyze the blood sugar content therein. If the blood sugar is too high he needs to inject insulin. In order to analyze his blood the user brings the detection unit 1 into contact with his blood. The detection unit 1 preferably automatically delivers an analysis of the blood sample with respect to certain components contained therein, e.g. the blood sugar amount. Those data are preferably stored in a data memory 4. Data gained by the detection unit 1 may then be displayed on the display unit 3, for example an LCD. On the display also former results, markings or advises stored in the memory 4 or fetched from a remote data bank via the transceiver 9 etc. may additionally be displayed. On the basis of the data displayed on the LCD, the user may take any decisions, if necessary. The telecommunication unit 5, may be programmed in a way that data stored in the data memory 4 is automatically transferred to a certain remote transceiver 9 connected to a medical data bank or a computer for example in a doctor's office. The transmission may be effected after a period of time has elapsed or a certain number of data acquisitions, i.e. taking of blood samples have been completed. The telecommunication unit 5 may be programmed such that a number corresponding to a certain internet address to which the data is to be transferred is automatically dialed. However, the user may also individually transfer data corresponding to one or several data acquisitions, i.e. blood samplings. In order to do this he may use the input unit 8 which may be designed as a keyboard or a key panel. The user may also use features such as a program menu displayed on the display unit 3 permitting to select certain functions and telephone numbers and furthermore a voice recognition unit capable of recognizing a spoken number or a name of a subscriber of a mobile telephone network or the internet. On dialing a number data is transferred to the specific destination, for example the doctor's office. At the same time, the user may call the doctor using the portable medical device which preferably also comprises a telecommunication unit, which also permits a voice communication. After having received the data the doctor will analyze those and decide whether or how the treatment needs to be changed.

The doctor will then communicate with his patient to provide him with his advise. Since the telecommunication unit of the portable medical device comprises a receiver for voice and/or data, either a direct conversation between the doctor and his patient is possible and/or written advises using SMS or email may be sent to the user. Finally if the portable medical device is equipped with a medical application unit or connected therewith the doctor may even remotely control an application unit which then provides the desired amount of medicine for the user. This may be effected by providing an amount of fluid or powder medicine or pills at an output of the application unit or by directly introducing the desired amount of fluid medicine e.g. in a syringe connected to the application unit.

Even though the present invention has been disclosed with respect to a specific embodiment concerning the analysis of blood samples in the case of a diabetes patient, the portable medical device may also be used with respect to the analysis of any other fluids or substances contained in the human body as well as for detecting other parameters concerning the condition of a human body such as temperature, blood pressure, action currents of the heart, the brain, etc.

The features of the invention disclosed in the claims, in the description and in the drawings may be significant for the realization of the invention either alone or in any combination thereof.

## Claims

1. Portable medical device which comprises a detection unit (1) for detecting at least one parameter concerning the condition of a living body; **characterized in that** it further comprises a telecommunication unit (5) for a data and/or voice communication, the telecommunication unit (5) being capable of transferring data to and/or of receiving data from a remote data transceiver (9).

2. Portable medical device according to claim 1, **characterized in that** said telecommunication unit (5) automatically transfers data to and/or receives data from said remote data transceiver (9).

3. Portable medical device according to claim 1 or 2, **characterized in that** said telecommunication unit (5) transfers data to and/or receives data from said remote data transceiver (9) in response to an instruction of a user, wherein said instruction is entered by an input unit (8) provided on the portable medical device.

4. Portable medical device according to one of the preceding claims, **characterized in that** it further comprises a display unit (3).

5. Portable medical device according to one of the preceding claims, **characterized in that** it includes a telecommunication unit (5) using a mobile telephone network.

6. Portable medical device according to one of the preceding claims, **characterized in that** the detection unit (1) is body fluid sampling unit, a body temperature measurement device, a blood pressure measurement device, a pulse measurement device, a device for measuring action currents of the heart or the brain or a combination of two ore more of them.

7. Portable medical device according to one of the preceding claims, **characterized in that** it comprises a medical application unit (2).

8. Portable medical device according to claim 7, **characterized in that** the medical application unit (2) is a syringe or a cardiac pacemaker.

9. Portable medical device according to claim 7 or 8, **characterized in that** the medical application unit (2) automatically provides a prescribed amount of medicine either in response to one or several parameters detected by the detection unit (1) or in response to an instruction received from said remote data transceiver (9) or in response to an instruction entered by the user.

10. A mobile phone comprising a portable medical analysis device according to one of the preceding claims.
